# EUROPEAN PATENT SPECIFICATION

(11) **EP 1 545 437 B1**
(45) Date of publication and mention of the grant of the patent: **15.12.2010**
(21) Application number: 03799037.1
(22) Date of filing: 01.10.2003
(51) Int. Cl.: A61K 8/11, A61Q 1/06, A61Q 1/10, A61Q 1/12

(54) **COMPOSITION INTENDED TO BE APPLIED TO THE SKIN AND THE INTEGUMENTS**
ZUSAMMENSETZUNG ZUM AUFTRAGEN AUF DIE HAUT UND DIE INTEGUMENTE
COMPOSITION DESTINEE A ETRE APPLIQUEE SUR LA PEAU ET LES TEGUMENTS

(30) Priority: 02.10.2002 FR 0212215; 25.11.2002 US 428723 P
(43) Date of publication of application: 29.06.2005
(73) Proprietor: L'Oréal, 75008 Paris (FR)
(72) Inventor: DUMOUSSEAUX, Christophe, Shinjuku, Tokyo (JP)
(74) Representative: Tanty, François
(86) International application number: PCT/IB2003/004306
(87) International publication number: WO 2004/030640

(56) References cited:
- EP-A- 1 184 426
- FR-A- 2 594 130
- US-A- 5 356 617
- PATENT ABSTRACTS OF JAPAN vol. 1999, no. 04, 30 avril 1999 (1999-04-30) & JP 11 012493 A (KOSE CORP), 19 janvier 1999 (1999-01-19)

## Description

The present invention relates to compositions intended to be applied to the skin, including mucous membranes, especially the lips, and the integuments, especially the nails, the eyelashes, the eyebrows and the hair.

It is known practice to incorporate organic pigments into cosmetic compositions, these pigments making it possible to obtain colours with high saturation. However, their covering power is poor, which leads to mineral pigments being added to the composition.

EP-1184426 (Toda Kogyo Corporation) describes the use of a paint/inkt composition comprising composite particles with an average particle size of 0,001-10,0 microns comprising a white mineral core of TiO2, coated with an organic blue pigment (i.e. Cl 74160= phthalocyanine blue) in an amount of from 1 to 500 weight% of said white core particles, and a binder selected from the organosilicon compounds, as liquid or solid compositions. Using the same binder, gives adherence to the surface without any covalent bonds. Oil-, solvent-or water-based compositions and additives make these compositions suitable for cosmetic applications, but no additives mentioned to prepare lipstick compositions.
Furthermore, JP-11012493 (Kose Corp) a cosmetic composite powder comprising a coloured mineral core (MgO, silicates) coated with an organic colorant and calcium chloride or aluminium chloride as binder is used.

The presence in the composition of a mixture of pigments of different nature entails a risk of variability of the properties, especially when different shades are produced by changing the proportions of organic and mineral pigments. This is because the behaviour of the organic and mineral pigments towards the other constituents of the composition is not the same, which results in difficulties of formulation. Thus, the sticks of a range of shades of lipsticks will have variable hardnesses.

Furthermore, the behaviour of the organic and mineral pigments towards the other constituents of the composition is liable to be not the same, which results in difficulties of formulation and a risk of modification of the makeup result over time, for example when a volatile compound evaporates. Thus, for example, certain lipsticks comprise mineral pigments such as TiO₂ and an oily phase; the TiO₂ particles may become white when they are no longer coated with the oily phase, which changes the colour of the composition applied and poses a problem of stability of the colour over time.

Finally, when lakes are used, the organic dye used in the lake is liable to transfer onto the support and stain it. The pigments conventionally used in cosmetic formulations are about one micrometre or larger in size. This large size, combined with a high density, results in sedimentation and stability problems in liquid formulations. It also prevents the production of transparency effects associated with a large saturation of the colour.

There is a need to benefit from a composition, especially a cosmetic composition, for solving all or some of the abovementioned drawbacks.

One subject of the invention is thus, among others, the use of a composition comprising, in a physiologically acceptable medium, at least one composite pigment comprising:
- a mineral core made of titanium oxide,
- at least one organic pigment at least partially covering the mineral core, and
- at least one binder chosen from the group consisting of silicone compounds, polymeric and oligomeric compounds or the like, and in particular from organosilanes, fluoroalkyl organosilanes and polysiloxanes, and couplers based on silane, on titanates, on aluminates or on zirconates, and mixtures thereof for fixing the organic pigment onto the mineral core,
for making up the skin, the lips or the integuments.

The invention makes it possible to benefit from cosmetic compositions comprising at least one composite pigment that has both relatively strong covering power and the advantages of an organic pigment, especially relatively high colour saturation. The binder can allow the organic pigment to fix onto the mineral core without any covalent bonds between the two.

The composite pigment may have a density higher than that of the organic pigment alone, on account of the presence of the mineral core, the density of the said core possibly being higher than that of the organic pigment.

A suitable shade may be obtained by mixing composite pigments according to the invention, or alternatively by mixing organic pigments into the composite pigment or with successive layers of binders and organic pigments in the composite pigment.

The invention may thus make it possible to prepare compositions without mixing organic and mineral pigments and/or not comprising any pigment having the sensitivity of lakes, which may facilitate the formulation and avoid the drawbacks associated with the use of lakes.

In addition, by appropriately selecting the organic pigment, and especially its colour, it is possible, where appropriate, to reinforce the colour of the mineral core. For example, it is possible to obtain a deep black, for example by combining a black iron oxide and an organic pigment. The size of the mineral core may be readily varied so as to modify the size of the final composite pigment. For example, coloured pigments of nanometric size may be obtained. These correctly dispersed nanometric-sized pigments make it possible especially to avoid the sedimentation problems observed on pigments of larger size. The refractive index of the composite pigment may also be readily modified by varying the index of the mineral core. The presence of the binder makes it possible both to fix the organic pigment onto the surface during the manufacture of the pigment, and to reduce any transfer of the pigment onto the support.

The term "physiologically acceptable medium" denotes a non-toxic medium that may be applied to human skin, lips or integuments. The physiologically acceptable medium will be adapted to the nature of the support onto which the composition is to be applied, and also to the form in which the composition is intended to be packaged, especially solid, semi-solid or fluid at room temperature and atmospheric pressure.

The term "cosmetic composition" denotes a composition as defined in Directive 93/35/EEC of the Council of 14 June 1993.

The organic pigment may be chosen from particulate compounds that are insoluble in the physiologically acceptable medium of the composition.

The organic pigment may be chosen especially from the nitroso, nitro, azo, xanthene, quinoline, anthraquinone, phthalocyanin and carmine families, and carbon black.

The pigment may be chosen from the following non-limiting list, which refers to the Color Index, as does Directive 93/35/EEC:
- blue pigment: CI 42090, 69800, 69825, 73000, 74100, 74160;
- yellow pigment: CI 11680, 11710, 15985, 19140, 20040, 21100, 21108, 47000,47005;
- green pigment: CI 61565, 61570, 74260;
- orange pigment: CI 11725, 15510, 45370, 71105;
- red pigment: CI 12085, 12120, 12370, 12420, 12490, 14700, 15525, 15580, 15620, 15630, 15800, 15850, 15865, 15880, 17200, 26100, 45380, 45410, 58000, 73360, 73915,75470;
- black pigment: CI 77266.

The binder may be of any type provided that it allows the organic pigment to adhere to the surface of the mineral core.

The binder is chosen especially from the list comprising silicone compounds, polymeric or oligomeric compounds or the like, and in particular from alkoxysilanes, fluoroalkylsilanes and polysiloxanes, and couplers based on silane, on titanates, on aluminates or on zirconates, and mixtures thereof.

As mentioned above, the mineral core may be coloured, i.e., for example, non-transparent, this core possibly being, for example, white or black.

The mass proportion of the organic pigment may be between 1 and 500 parts by weight per 100 parts by weight of the core, for example.

The size of the composite pigment particles may be between 0.001 and 150 µm, especially between 0.01 and 50 µm, for example between 0.01 and 25 µm or between about 0.05 µm and about 10 µm. The size of the pigment particles may especially be less than 2 µm, or even less than or equal to 1 µm. The term "size" denotes the dimension given by the statistical particle-size distribution to half of the population, known as the D50.

Out of concern for aesthetics, it is generally preferable for the composite pigment particles to be not at all perceptible, or not readily perceptible, to the naked eye, at the surface of the composition applied to its support. It is also desirable for the composite pigment particles not to have sizes such that they create a sensation of discomfort on the support. The use of particles less than or equal to 250 µm and better still less than or equal to 150 µm in size, for example less than 15 µm, is thus preferred. The particle size may also depend on the nature of the support onto which the composition is intended to be applied; certain parts of the body or face may, for example, tolerate larger sizes more readily than others without experiencing discomfort.

The composite pigment particles may have varied forms. These particles may especially be in the form of platelets or globules, in particular in spherical form, and may be hollow or solid. The expression "in the form of platelets" denotes particles in which the ratio of the largest dimension to the thickness is greater than or equal to 5.

The composition may comprise only composite pigments as defined above or, as a variant, comprise composite pigments and also pigments having another structure, especially mineral pigments, interference pigments, lakes or organic pigments. The composition may especially be free of uncoated TiO₂ particles.

The composition may comprise one or more composite pigments in a mass proportion that may be between 0.1% and 20%, in particular between 0.1% and 15% and better still between 0.5% and 10%, especially by weight relative to the total weight of the composition.

The composition may comprise at least one aqueous or organic solvent.

When the composition comprises one or more organic solvents, these solvents may be present in an amount ranging from 0 to 99% relative to the total weight of the composition.

In general, the amount of solvent(s), especially organic solvent(s), will depend on the nature of the support onto which the composition is intended to be applied.

In the case of a nail varnish, for example, the organic solvent may be present in the composition in a content ranging, for example, from 30% to 99% by weight and preferably from 60% to 90% by weight relative to the total weight of the composition.

The composition may comprise at least one organic solvent chosen from the following list:
- ketones that are liquid at room temperature, such as methyl ethyl ketone, methyl isobutyl ketone, diisobutyl ketone, isophorone, cyclohexanone or acetone;
- alcohols that are liquid at room temperature, such as ethanol, isopropanol, diacetone alcohol, 2-butoxyethanol or cyclohexanol;
- glycols that are liquid at room temperature, such as ethylene glycol, propylene glycol, pentylene glycol or glycerol;
- propylene glycol ethers that are liquid at room temperature, such as propylene glycol monomethyl ether, propylene glycol monomethyl ether acetate or dipropylene glycol mono-n-butyl ether;
- short-chain esters (containing from 3 to 8 carbon atoms in total) such as ethyl acetate, methyl acetate, propyl acetate, n-butyl acetate or isopentyl acetate;
- alkanes that are liquid at room temperature, such as decane, heptane, dodecane or cyclohexane.

The composition may also comprise water or a mixture of water and hydrophilic organic solvents commonly used in cosmetics, for instance alcohols and especially linear or branched lower monoalcohols containing from 2 to 5 carbon atoms, for instance ethanol, isopropanol or n-propanol, polyols, for instance glycerol, diglycerol, propylene glycol, sorbitol, pentylene glycol or polyethylene glycols. The composition may also contain hydrophilic C₂ ethers and C₂-C₄ aldehydes. Water or a mixture of water and of hydrophilic organic solvents may be present in the composition in a content ranging, for example, from 0% to 90%, especially 0.1% to 90% by weight, preferably from 0% to 60% by weight and especially 0.1% to 60% by weight, relative to the total weight of the composition.

The composition, especially when it is intended to be applied to the lips, may comprise a fatty phase and especially at least one fatty substance that is liquid at room temperature (25°C) and/or a fatty substance that is solid at room temperature, such as waxes, pasty fatty substances, and gums, and mixtures thereof. The fatty phase may also contain lipophilic organic solvents.

The composition may have, for example, a continuous fatty phase, which may contain less than 5% water and especially less than 1% water relative to its total weight, and in particular may be in anhydrous form.

As fatty substances that are liquid at room temperature, often referred to as "oils", mention may be made of: hydrocarbon-based plant oils such as liquid triglycerides of fatty acids of 4 to 10 carbon atoms, for instance heptanoic or octanoic acid triglycerides, or sunflower oil, maize oil, soybean oil, grapeseed oil, sesame seed oil, apricot oil, macadamia oil, castor oil, avocado oil, caprylic/capric acid triglycerides, jojoba oil or shea butter; linear or branched hydrocarbons of mineral or synthetic origin, such as liquid paraffins and derivatives thereof, petroleum jelly, polydecenes, and hydrogenated polyisobutene such as parleam; synthetic esters and ethers, especially of fatty acids, for instance purcellin oil, isopropyl myristate, 2-ethylhexyl palmitate, 2-octyldodecyl stearate, 2-octyldodecyl erucate or isostearyl isostearate; isononyl isonanoate; hydroxylated esters, for instance isostearyl lactate, octyl hydroxystearate, octyldodecyl hydroxystearate, diisostearyl malate, triisocetyl citrate or fatty alkyl heptanoates, octanoates and decanoates; polyol esters, for instance propylene glycol dioctanoate, neopentyl glycol diheptanoate or diethylene glycol diisononanoate; and pentaerythritol esters; fatty alcohols containing from 12 to 26 carbon atoms, for instance octyldodecanol, 2-butyloctanol, 2-hexyldecanol, 2-undecylpentadecanol or oleyl alcohol; partially hydrocarbon-based and/or silicone-based fluoro oils; silicone oils, for instance volatile or non-volatile, linear or cyclic polymethylsiloxanes (PDMSs) that are liquid or pasty at room temperature, for instance cyclomethicones, dimethicones, optionally comprising a phenyl group, for instance phenyl trimethicones, phenyltrimethylsiloxydiphenylsiloxanes, diphenylmethyldimethyltrisiloxanes, diphenyl dimethicones, phenyl dimethicones and polymethylphenylsiloxanes; and mixtures thereof. The oils may be present in a content ranging from 0.01 % to 90% and better still from 0.1 % to 85% by weight, relative to the total weight of the composition.

The pasty fatty substances are generally hydrocarbon-based compounds with a melting point of between 25 and 60°C and preferably between 30 and 45°C, and/or a hardness of between 0.001 and 0.5 MPa and preferably between 0.005 and 0.4 MPa, for instance lanolins and derivatives thereof.

The waxes may be solid at room temperature (25°C), with a reversible solid/liquid change of state, having a melting point of greater than 30°C which may be up to 200°C, a hardness of greater than 0.5 MPa, and having in solid form an anisotropic crystal organization. In particular, the waxes may have a melting point of greater than 25°C and better still greater than 45°C. The waxes may be hydrocarbon-based waxes, fluoro waxes and/or silicone waxes and may be of plant, mineral, animal and/or synthetic origin. As waxes that may be used, mention may be made of beeswax, carnauba wax or candelilla wax, paraffin, microcrystalline waxes, ceresin or ozokerite; synthetic waxes, such as polyethylene wax or Fischer-Tropsch wax, and silicone waxes, for instance alkyl- or alkoxydimethicones containing from 16 to 45 carbon atoms. The composition may contain from 0 to 50% by weight of waxes, or even from 1% to 30% by weight of waxes, relative to the total weight of the composition.

The gums that may be used are generally high molecular weight polydimethylsiloxanes (PDMSs) or cellulose gums or polysaccharides.

The composition may also comprise, for example, a film-forming polymer, especially in the case of a mascara or a nail varnish. The term "film-forming polymer" denotes a polymer capable, by itself or in the presence of an auxiliary film-forming agent, of forming a continuous film that adheres to a support and especially to keratin materials.

Among the film-forming polymers that may be used in a composition according to the invention, mention may be made, inter alia, of synthetic polymers, of free-radical type or of polycondensate type, polymers of natural origin, such as nitrocellulose or cellulose esters, and mixtures thereof.

The film-forming polymers of free-radical type may especially be vinyl polymers or copolymers, especially acrylic polymers.

The vinyl film-forming polymers may result from the polymerization of ethylenically unsaturated monomers containing at least one acid group and/or esters of these acidic monomers and/or amides of these acidic monomers, for instance α,β-ethylenic unsaturated carboxylic acids such as acrylic acid, methacrylic acid, crotonic acid, maleic acid or itaconic acid.

The vinyl film-forming polymers may also result from the homopolymerization or copolymerization of monomers chosen from vinyl esters, for instance vinyl acetate, vinyl neodecanoate, vinyl pivalate, vinyl benzoate and vinyl t-butylbenzoate, and styrene monomers, for instance styrene and α-methylstyrene.

Among the film-forming polycondensates that may be mentioned are polyurethanes, polyesters, polyesteramides, polyamides and polyureas, this list not being limiting.

The optionally modified polymers of natural origin may be chosen from shellac resin, sandarac gum, dammar resins, elemi gums, copal resins, cellulose-based polymers, such as nitrocellulose, ethylcellulose or nitrocellulose esters chosen, for example, from cellulose acetate, cellulose acetobutyrate and cellulose acetopropionate, and mixtures thereof.

The film-forming polymer may be present in the form of particles in aqueous or oily dispersion, generally known as latices or pseudolatices. The film-forming polymer may comprise one or more stable dispersions of particles of generally spherical polymers of one or more polymers, in a physiologically acceptable liquid fatty phase. These dispersions are generally known as polymer NADs (Non-Aqueous Dispersions), as opposed to latices, which are aqueous polymer dispersions. These dispersions may especially be in the form of polymer nanoparticles in stable dispersion in the said fatty phase. The nanoparticles are preferably between 5 and 600 nm in size. The techniques for preparing these dispersions are well known to those skilled in the art.

Aqueous dispersions of film-forming polymers that may be used include the acrylic dispersions sold under the names Neocryl XK-90®, Neocryl A-1070®, Neocryl A-1090®, Neocryl BT-62®, Neocryl A-1079® and Neocryl A-523® by the company Avecia-Neoresins, Dow Latex 432® by the company Dow Chemical, Daitosol 5000 AD® by the company Daito Kasei Kogyo; or the aqueous polyurethane dispersions sold under the names Neorez R-981® and Neorez R-974® by the company Avecia-Neoresins, Avalure UR-405®, Avalure UR-410®, Avalure UR-425®, Avalure UR-450®, Sancure 875®, Sancure 861®, Sancure 878® and Sancure 2060® by the company Goodrich, Impranil 85® by the company Bayer and Aquamere H-1511® by the company Hydromer; the sulphopolyesters sold under the brand name Eastman AQ by the company Eastman Chemical Products.

The composition according to the invention may also comprise an auxiliary film-forming agent that promotes the formation of a film with the film-forming polymer.

The composition may also comprise fillers. The term "fillers" denotes particles of any form, which are insoluble in the medium of the composition, irrespective of the temperature at which the composition is manufactured. These fillers may serve especially to modify the rheology or texture of the composition. The nature and amount of the solid substances depend on the desired mechanical properties and textures.

Examples of fillers that may be mentioned, inter alia, include talc, mica, silica, kaolin, sericite, polyamide powder, polyethylene powder, polytetrafluoroethylene powder, polymethyl methacrylate powder, polyurethane powder, starch powders and silicone resin beads.

The composition may comprise at least one cosmetic or dermatological active agent. As cosmetic, dermatological, hygiene or pharmaceutical active agents that may be used in the compositions of the invention, mention may be made of moisturizers (polyols, for instance glycerol), vitamins (C, A, E, F, B or PP), essential fatty acids, essential oils, ceramides, sphingolipids, liposoluble sunscreens or sunscreens in the form of nanoparticles, and specific skin-treating active agents (protective agents, antibacterial agents, anti-wrinkle agents, etc.). These active agents may be used, for example, in concentrations of from 0 to 20% and especially from 0.001 to 15% relative to the total weight of the composition.

The cosmetic composition may also contain ingredients commonly used in cosmetics, for instance thickeners, surfactants, trace elements, moisturizers, softeners, sequestering agents, fragrances, acidifying or basifying agents, preserving agents, antioxidants, UV-screening agents or dyes, or mixtures thereof.

The cosmetic composition may also comprise, depending on the type of application envisaged, constituents conventionally used in the fields under consideration, which are present in an amount that is suitable for the desired presentation form.

The composition may be in various forms, depending on its intended use. The cosmetic composition may thus be in any presentation form normally used for topical application and especially in anhydrous form, in the form of an oily or aqueous solution, an oily or aqueous gel, an oil-in-water, water-in-oil, wax-in-water or water-in-wax emulsion, a multiple emulsion, or a dispersion of oil in water by means of vesicles located at the oil/water interface.

The composition may be in the form of a cast product, especially in the form of a stick in the case of a lipstick or a lipcare product.

The composition may also be in various other forms, for example in the form of a more or less viscous liquid, a gel or a paste.

The composition may also be in the form of a semi-solid or a solid, for example a cake to be moistened at the time of use, so as to allow it to be disintegrated.

The cosmetic composition may constitute, inter alia, a lipstick, a liquid gloss, a lipstick paste, a makeup rouge, a lip pencil, a solid or fluid foundation, a concealer product or eye-contour product, an eyeliner, a mascara, a nail varnish, an eyeshadow, a body or hair makeup product or an antisun product or skin-colouring product.

A subject of the invention is thus also a lipstick comprising a composition as defined above.

### COMPOSITE PIGMENT

The composite pigment according to the invention may be prepared, for example, by one of the processes described in European patent applications EP 1 184 426 and EP 1 217 046.

A composite pigment according to the invention is composed especially of particles comprising:
- a mineral core made of titanium oxide,
- at least one binder chosen from the group consisting of silicone compounds, polymeric and oligomeric compounds or the like, and in particular from organosilanes, fluoroalkyl organosilanes and polysiloxanes, and couplers based on silane, on titanates, on aluminates or on zirconates, and mixtures thereof for fixing the organic pigments onto the core, and
- at least one organic pigment at least partially covering the core.

The composite pigments may have, for example, a BET specific surface area of between 0.5 and 500 m²/g, especially 1.5 to 400 m²/g, and in particular 2 to 300 m²/g. The "BET specific surface area" is the value measured by the BET method.

Use of a composition comprising, in a physiologically acceptable medium, at least one composite pigment comprising:
a mineral core made of titanium oxide,
at least one organic pigment at least partially covering the mineral core, and
at least one binder chosen from the group consisting of silicone compounds, polymeric and oligomeric compounds or the like, and in particular from organosilanes, fluoroalkyl organosilanes and polysiloxanes, and couplers based on silane, on titanates, on aluminates or on zirconates, and mixtures thereof for fixing the organic pigment onto the mineral core, for making up the skin, the lips or integuments.

More specifically as lipstick comprising these composite pigments.

### MINERAL CORE

This may be in any form that is suitable for fixing organic pigment particles, for example spherical, granular, polyhedral, acicular, fusiform, in the form of flakes, grains of rice or scales, and also a combination of these forms.

Preferably, the ratio of the largest dimension to the smallest dimension is between 1 and 50.

The mean size may be, for example, between 0.0009 µm and 9.95 µm, especially 0.002 µm and 9.45 µm, or even between 0.009 µm and 8.95 µm. These size values are most particularly suitable when the composite pigment particles are prepared according to the processes described in the abovementioned patents EP 1 184 426 and EP 1 217 046. The term "mean size" denotes the mean value obtained by measuring 350 mineral cores by micrography.

The mineral cores may have a BET specific surface area of between 0.5 and 500 m²/g, especially between 1 and 400 m²/g, and in particular between 1.5 and 300 m²/g.

When the mineral cores are white, their covering power may be greater than 600 cm²/g. When the said mineral cores are not white, i.e. when they are coloured, transparent or black, their covering power may be less than 600 cm²/g.

### BINDER

The binder may be of any type provided that it allows the organic pigment to adhere to the surface of the mineral core.

The binder is chosen from a list comprising silicone compounds, polymeric or oligomeric compounds or the like, and in particular from organosilanes, fluoroalkyl organosilanes and polysiloxanes, and couplers based on silanes, on titanates, on aluminates or on zirconates, and mixtures thereof.

The silicone compound may be chosen from a non-limiting list especially comprising:
- the organosilanes (1) obtained from alkoxysilanes,
- the modified or unmodified polysiloxanes (2) chosen from a non-limiting list comprising:
   - the modified polysiloxanes (2A) comprising at least one radical chosen especially from polyethers, polyesters and epoxy compounds (these will be referred to as "modified polysiloxanes"),
   - the polysiloxanes (2B) bearing, on a silicon atom located at the end of the polymer, at least one group chosen from a non-limiting list comprising carboxylic acids, alcohols and hydroxyl groups, and
- the fluoroalkyl organosilane compounds (3) obtained from fluoroalkylsilanes.

The organosilane compounds (1) may be obtained from alkoxysilane compounds represented by formula (I):

R¹ₐ Si X₄₋ₐ (I)

in which:
- R¹ represents C₆H₅-, (CH₃)₂ CH CH₂- or n- C_{b} H_{2b+1}- (in which b ranges from 1 to 18),
- X represents CH₃O- or C₂H₅O-, and
- a ranges from 0 to 3.

Specific examples of alkoxysilane compounds may include alkoxysilanes chosen from: methyltriethoxysilane, dimethyldiethoxysilane, phenyltriethyoxysilane, diphenyldiethoxysilane, methyltrimethoxysilane, dimethyldimethoxysilane, phenyltrimethoxysilane, diphenyldimethoxysilane, isobutyltrimethoxysilane, decyltrimethoxysilane and the like, in particular from methyltriethoxysilane, phenyltriethyoxysilane, methyltrimethoxysilane, dimethyldimethoxysilane and isobutyltrimethoxysilane, and even better still methyltriethoxysilane, methyltrimethoxysilane or phenyltriethyoxysilane.

The polysiloxanes (2) may especially correspond to formula (II): 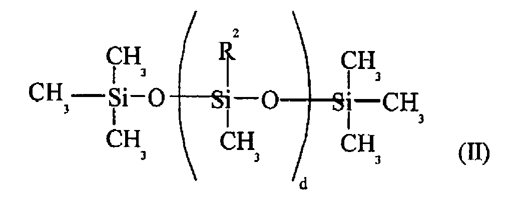 in which R² represents H- or CH₃- and d ranges from 15 to 450.

Among these polysiloxanes, those for which R² represents H are preferred.

The modified polysiloxanes (2A) may especially correspond to the following formulae:
- (a¹) modified polysiloxanes bearing polyethers, represented by formula (III) 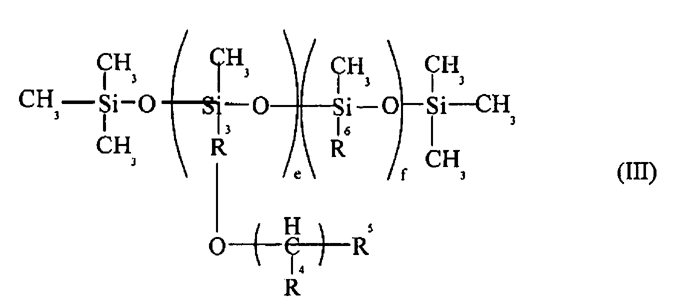 in which R³ represents -(CH₂)ₕ-; R⁴ represents -(CH₂)ᵢ- CH₃; R⁵ represents -OH, -COOH, -CH = CH₂, -C (CH₃) = CH₂ or -(CH₂)ⱼ- CH₃ ; R⁶ represents -(CH2)ₖ- CH₃; g and h ranging independently from 1 to 15; j and k ranging independently from 0 to 15; and e ranging from 1 to 50 and f ranging from 1 to 300,
- (a²) modified polysiloxanes bearing polyesters, represented by formula (IV): 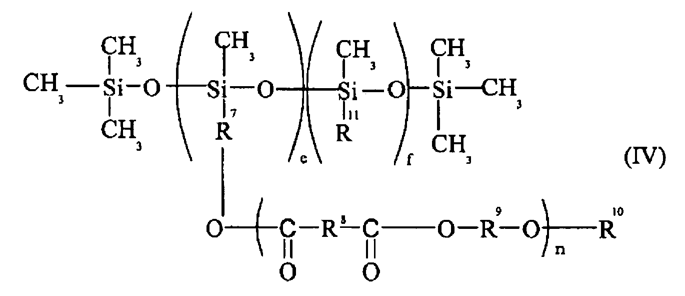 in which R⁷, R⁸ and R⁹ independently represent -(CH₂)_{q}-; R¹⁰ represents -OH; -COOH, -CH = CH₂, -C(CH₃) = CH₂ or -(CH₂)ᵣ- CH₃; R¹¹ represents -(CH₂)₅- CH₃ ; n and q ranging independently from 1 to 15, r and s ranging independently from 0 to 15; e ranging from 1 to 50 and f ranging from 1 to 300,
- (a³) modified polysiloxanes bearing epoxy radicals represented by formula (V): 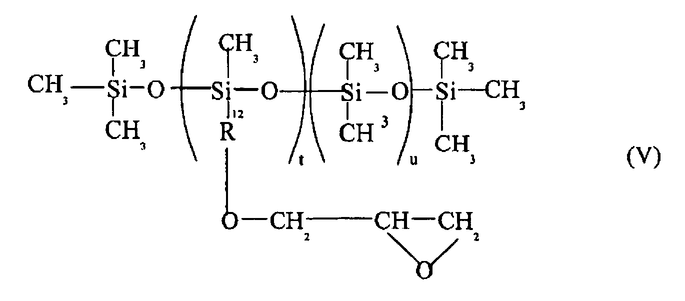 in which R¹² represents -(CH₂)ᵥ-; v ranging from 1 to 15; t ranging from 1 to 50 and u ranging from 1 to 300; or mixtures thereof.
Among the modified polysiloxanes (2A), the modified polysiloxanes bearing polyethers of formula (III) are preferred.
The polysiloxanes modified on the end portion (2B) may correspond to formula (VI): 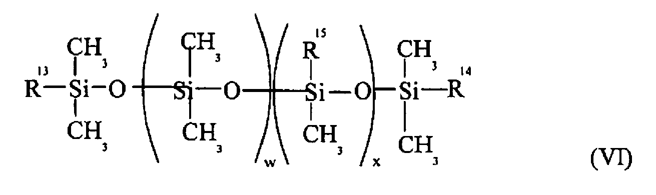 in which R¹³ and R¹⁴ may represent -OH, R¹⁶ OH or R¹⁷ COOH, independently of each other; R¹⁵ represents -CH₃ or -C₆H₅; R¹⁶ and R¹⁷ represent -(CH₂)_{y}-; y ranging from 1 to 15; w ranging from 1 to 200 and x ranging from 0 to 100.

Among these polysiloxanes modified on at least one end, those bearing at least radical (R¹⁶ and/or R¹⁷) bearing a carboxylic acid group on at least one terminal silicon atom are more preferred.

The fluoroalkyl organosilane compounds (3) may be obtained from fluoroalkyl silanes represented by formula (VII):

CF₃(CF₂)_{z}CH₂CH₂ (R¹⁸)ₐSiX₄₋ₐ (VII)

in which:
- R¹⁸ represents CH₃-, C₂H₅-, CH₃O- or C₂H₅O-,
- X represents CH₃O- or C₂H₅O-,
- Z ranges from 0 to 15 and a ranges from 0 to 3.

The fluoroalkyl silanes may be chosen especially from a non-limiting list especially comprising trifluoropropyltrimethoxysilane, tridecafluorooctyltrimethoxysilane, heptadecafluorodecyltrimethoxysilane, heptadecafluorodecylmethyldimethoxysilane, trifluoropropyltriethoxysilane, tridecafluorooctyltriethoxysilane, heptadecafluorodecyltriethoxysilane, heptadecafluorodecylmethyldiethoxysilane and the like, in particular trifluoropropyltrimethoxysilane, tridecafluorooctyltrimethoxysilane and heptadecafluorodecyltrimethoxysilane, and even better still trifluoropropyltrimethoxysilane and tridecafluorooctyltrimethoxysilane.

The silane-based couplers may be chosen from a non-limiting list especially comprising vinyltrimethoxysilane, vinyltriethoxysilane, γ-aminopropyltriethoxysilane, γ-glycidoxypropyltrimethoxysilane, γ-mercaptopropyltrimethoxysilane, γ-methacryloxypropyltrimethoxysilane, N-β-(aminoethyl)-γ-aminopropyltrimethoxysilane, γ-glycidoxypropylmethyldimethoxysilane and γ-chloropropyltrimethoxysilane, and the like.

The titanate-based couplers may be chosen from the list comprising isopropylstearoyl titanate, isopropyltris(dioctyl pyrophosphate) titanate, isopropyltris(N-aminoethylaminoethyl) titanate, tetraoctylbis(ditridecyl phosphate) titanate, tetrakis(2,2-diaryloxymethyl-1-butyl)bis(ditridecyl)phosphate titanate, bis(dioctyl pyrophosphate)oxyacetate titanate and bis(dioctyl pyrophosphate)ethylene titanate, and the like.

The aluminate-based couplers may be chosen from acetoalkoxyaluminium diisopropoxide, aluminium diisopropoxymonoethylacetoacetate, aluminium trisethylacetoacetate and aluminium trisacetylacetonate, and the like.

The zirconate-based couplers may be chosen from a list especially comprising zirconium tetrakisacetylacetonate, zirconium dibutoxybisacetylacetonate, zirconium tetrakisethylacetoacetate, zirconium tributoxymonoethylacetoacetate and zirconium tributoxyacetylacetonate, and the like.

The compounds used as binder may especially have a molecular mass that may range between 300 and 100 000.

In order to obtain a coat that covers the mineral cores uniformly, the binder is preferably in a form that is liquid or soluble in water or in various solvents.

The amount of binder may range from 0.01 to 15%, especially from 0.02% to 12.5% and in particular from 0.03 to 10% by weight (calculated relative to C or Si) relative to the weight of the particles comprising the core and the binder. For further details regarding the way of calculating the relative amount of the binder, reference may be made to patent application EP 1 184 426 A2.

### ORGANIC PIGMENT

The organic pigment may be chosen, for example, from aniline black, azo yellow, quinacridone, carmine, phthalocyanin blue, sorghum red, blue pigment: CI 42090, 69800, 69825, 73000, 74100, 74160; yellow pigment: CI 11680, 11710, 15985, 19140, 20040, 21100, 21108, 47000, 47005; green pigment: CI 61565, 61570, 74260; orange pigment: CI 11725, 15510, 45370, 71105; red pigment: CI 12085, 12120, 12370, 12420, 12490, 14700, 15525, 15580, 15620, 15630, 15800, 15850, 15865, 15880, 17200, 26100, 45380, 45410, 58000, 73360, 73915, 75470; black pigment: CI 77266.

The mass proportion of the organic pigment may be, for example, between 1 and 500 parts by weight, especially 1 and 400 parts by weight, better still 1 and 300 parts by weight, even better still 1 to 200 parts by weight, in particular 1 to 100 parts by weight, or even 1 to 75 parts by weight, for example 1 to 50 parts by weight, per 100 parts by weight of mineral core.

### PREPARATION OF THE COMPOSITE PIGMENT

The composite pigment may advantageously be prepared by the method described in patent application EP 1 184 426 A2.

To begin with, the particles intended to make up the mineral core are mixed with the binder.

In order for the binder to adhere uniformly to the surface of the mineral core, it is preferable to pretreat these particles in a mill, so as to disintegrate them.

The mixing and stirring conditions are chosen such that the core is uniformly covered with binder. These conditions may be regulated such that the linear charge is between 19.6 and 19 160 N/cm, in particular between 98 and 14 170 N/cm and better still between 147 and 980 N/cm; the treatment time is especially between 5 minutes and 24 hours and better still from 10 minutes to 20 hours; the spin speed may be between 2 and 1000 rpm, in particular between 5 and 1000 rpm and better still between 10 and 800 rpm.

After the binder has covered the mineral core, the organic pigment is added and mixed with stirring to adhere to the layer of binder.

The addition methods may be, for example, an addition in large amount, continuously or in small amount.

The mixing and stirring, whether they are mineral cores with the binder or organic pigment with the mineral cores covered with binder, may be performed using a machine capable of applying a spatular and/or compressive shearing force to the powder mixture. Such machines are, for example, wheel blenders, blade blenders and the like. Wheel blenders are most particularly suitable. A list of machines that may be suitable is given in patent application EP 1 184 426 A2.

### EXAMPLES

Cosmetic compositions comprising composite pigments with the formulations below may be prepared, these compositions being prepared according to the preparation processes conventionally used in cosmetics.

### Example 1: Nail varnish (solvent-based)

| | |
|---|---|
| Nitrocellulose | 19% |
| N ethyl o,p toluenesulphonamide | 6% |
| Tributyl acetyl citrate | 6% |
| Rheology agent (hectorite) | 1.2% |
| Composite pigment(s) according to the invention | 2% |
| Isopropanol | 8% |
| Ethyl acetate / butyl acetate | qs 100% |

### Example 2: Nail varnish (water-based)

| | |
|---|---|
| Latex (PU, 35% solids content) | 72.5% |
| Gelling agent (Laponite XLS) | 1.2% |
| Composite pigment(s) according to the invention | 1% |
| Water | qs 100% |

### Example 3: Lipstick

| | |
|---|---|
| Microcrystalline wax | 2% |
| Ozokerite | 5% |
| Candelilla wax | 7% |
| Carnauba wax | 3% |
| Capric/caprylic acid triglycerides | 18% |
| Octyldodecanol | 10% |
| Lanolin oil | 6% |
| Acetylated lanolin oil | 6% |
| Composite pigment(s) according to the invention | 9% |
| Fragrance | 0.5% |
| Castor oil | qs 100% |

### Example 4: Foundation

### Oily phase

- Surfactant sold under the trade name
   "Abil WE 09" by the company Goldschmidt 8%
- Cyclomethicone 23%
- Isododecane 10%
- Composite pigment(s) according to the invention 10%
- Nylon powder 5%

### Aqueous phase

- Demineralized water 42%
- Magnesium sulphate 1%
- Preserving agents 1%

### Example 5: Mascara

- Paraffin wax 2%
- Carnauba wax 4%
- Beeswax 8%
- Polylvinyl laurate (Mexomer PP from Chimex) 0.8%
- Vinyl acetate/allyl stearate copolymer (65/3 5) 2%
- Rice starch 1%
- Bentone 4%
- Propylene carbonate 2%
- Composite pigment(s) according to the invention 4%
- Preserving agents qs
- Isododecane qs 100%

### Example 6: Hair dye

- Composite pigment(s) according to the invention 0.5%
- Hydroxylethylcellulose 0.768%
- Nonionic surfactant: Alkyl (50/50 C8/C10) polyglucoside as an aqueous 60% solution 6%
- Benzyl alcohol 8%
- Polyethylene glycol (8EO) 12%
- Aqueous ammonia solution qs pH 9%
- Preserving agents qs
- Demineralized water qs 100%

A composite pigment comprising a titanium dioxide mineral core is used, the organic pigment being azo yellow and the binder methyltriethoxysilane.

Needless to say, the invention is not limited to the working examples that have just been given.

It is especially possible to use composite pigments according to the invention to prepare cosmetic compositions having formulations other than those given above.

The composite pigment may also be used to colour a dermatological composition.

Throughout the description, including the claims, the term "comprising one" should be understood as being synonymous with "comprising at least one", unless the opposite is specified.

The ranges given should be understood as being inclusive of the limits, unless the opposite is specified.

## Claims

1. Use of a composition comprising, in a physiologically acceptable medium, at least one composite pigment comprising:
- a mineral core made of titanium oxide,
- at least one organic pigment at least partially covering the mineral core, and
- at least one binder chosen from the group consisting of silicone compounds, polymeric and oligomeric compounds or the like, and in particular from organosilane, fluoroalkyl organosilanes and polysiloxanes, and couplers based on silane, on titanates, on aluminates or on zirconates, and mixtures thereof for fixing the organic pigment onto the mineral core,
for making up the skin, the lips or the integuments.

2. Use according to Claim 1, **characterized in that** the organic pigment is chosen from the group consisting of the following pigments (listed according to their Color Index): blue pigment: CI 42090, 69800, 69825, 73000, 74100, 74160; yellow pigment: CI 11680, 11710, 15985, 19140, 20040, 21100, 21108, 47000, 47005; green pigment: CI 61565, 61570, 74260; orange pigment: CI 11725, 15510, 45370, 71105; red pigment: CI 12085, 12120, 12370, 12420, 12490, 14700, 15525, 15580, 15620, 15630, 15800, 15850, 15865, 15880, 17200, 26100, 45380, 45410, 58000, 73360, 73915, 75470; black pigment: CI 77266.

3. Use according to any one of the preceding claims, **characterized in that** the mineral core is coloured.

4. Use according to any one of the preceding claims, **characterized in that** the mass proportion of the composite pigment in the composition is between 0.1% and 20%, especially between 0.1% and 15% and better still between 0.5% and 10% by weight relative to the total weight of the composition.

5. Use according to any one of the preceding claims, **characterized in that** the composition is free of uncoated TiO₂ particles.

6. Use according to any one of the preceding claims, **characterized in that** the size of the composite pigment particles is between 0.001 and 150 µm, preferably 0.01 and 50 µm, better still between 0.01 and 25 µm and even better still between 0.05 and 10 µm.

7. Use according to Claim 8, **characterized in that** the composite pigment particles are less than 2 µm in size.

8. Use according to Claim 7, **characterized in that** the composite pigment particles are less than or equal to 1 µm in size.

9. Use according to any one of the preceding claims, **characterized in that** the density of the mineral core is higher than that of the organic pigment.

10. Use according to Claim 1, **characterized in that** the density of the composite pigment is higher than that of the organic pigment.

11. Use according to Claim 1, **characterized in that** the organic pigment is fixed without any covalent bonds onto the mineral core.

12. Use according to any one of the preceding claims, **characterized in that** the composition comprises at least one cosmetic or dermatological active agent.

13. Use according to any one of the preceding claims, **characterized in that** the composition comprises at least one fatty substance, a wax, a gum or a film-forming polymer.

14. Use according to any one of the preceding claims, **characterized in that** the composition is in a solid or semi-solid form.

15. Use according to any one of the preceding claims, **characterized in that** the composition is in liquid, pasty or gelled form.

16. Lipstick comprising, in a physiologically acceptable medium, at least one composite pigment comprising:
- a mineral core made of titanium oxide,
- at least one organic pigment at least partially covering the mineral core, and
- at least one binder chosen from from the group consisting of silicone compounds, polymeric and oligomeric compounds or the like, and in particular from organosilanes, fluoroalkyl organosilanes and polysiloxanes, and couplers based on silane, on titanates, on aluminates or on zirconates, and mixtures thereof, for fixing the organic pigment onto the mineral core.

## Patentansprüche

1. Verwendung einer Zusammensetzung, umfassend in einem physiologisch verträglichen Medium mindestens ein Kompositpigment, umfassend:
- einen Mineralkern, hergestellt aus Titanoxid,
- mindestens ein organisches Pigment, welches mindestens teilweise den Mineralkem bedeckt, und
- mindestens ein Bindemittel, welches aus der Gruppe ausgewählt ist, die besteht aus Silikonverbindungen, polymeren und oligomeren Verbindungen oder dergleichen, und insbesondere von Organosilanen, Fluoralkylorganosilanen und Polysiloxanen, und Kupplungsmitteln, basierend auf Silan, auf Titanaten, auf Aluminaten oder auf Zirkonaten, und Gemischen davon, zum Fixieren des organischen Pigments auf dem Mineralkern,
zum Schminken der Haut, der Lippen oder der Integumente.

2. Verwendung gemäß Anspruch 1, **dadurch gekennzeichnet, dass** das organische Pigment aus der Gruppe ausgewählt ist, welche aus den folgenden Pigmenten (aufgelistet gemäß ihrem Farbindex) besteht: Blaupigment: CI 42090, 69800, 69825, 73000, 74100, 74160; Gelbpigment: CI 11680, 11710, 15985, 19140, 20040, 21100, 21108, 47000, 47005; Grünpigment: CI 61565, 61570, 74260; Orangepigment: CI 11725, 15510, 45370, 71105; Rotpigment: CI 12085, 12120, 12370, 12420, 12490, 14700, 15525, 15580, 15620, 15630, 15800, 15850, 15865, 15880, 17200, 26100, 45380, 45410, 58000, 73360, 73915, 75470; Schwarzpigment: CI 77266.

3. Verwendung gemäß einem der vorhergehenden Ansprüche, **dadurch gekennzeichnet, dass** der Mineralkern farbig ist.

4. Verwendung gemäß einem der vorhergehenden Ansprüche, **dadurch gekennzeichnet, dass** der Massenanteil des Kompositpigments in der Zusammensetzung zwischen 0,1 Gew.-% und 20 Gew.-%, insbesondere zwischen 0,1 Gew.-% und 15 Gew.-% und noch besser zwischen 0,5 Gew.-% und 10 Gew.-%, relativ zum Gesamtgewicht der Zusammensetzung, liegt.

5. Verwendung gemäß einem der vorhergehenden Ansprüche, **dadurch gekennzeichnet, dass** die Zusammensetzung frei von nicht-überzogenen TiO₂-Teilchen ist.

6. Verwendung gemäß einem der vorhergehenden Ansprüche, **dadurch gekennzeichnet, dass** die Größe der Kompositpigmentteilchen zwischen 0,001 und 150 µm, bevorzugt 0,01 und 50 µm, noch besser zwischen 0,01 und 25 µm und noch viel besser zwischen 0,05 und 10 µm liegt.

7. Verwendung gemäß Anspruch 6, **dadurch gekennzeichnet, dass** die Kompositpigmentteilchen eine Größe von weniger als 2 µm aufweisen.

8. Verwendung gemäß Anspruch 7, **dadurch gekennzeichnet, dass** die Kompositpigmentteilchen eine Größe von weniger als oder gleich 1 µm aufweisen.

9. Verwendung gemäß einem der vorhergehenden Ansprüche, **dadurch gekennzeichnet, dass** die Dichte des Mineralkerns höher ist als die des organischen Pigments.

10. Verwendung gemäß Anspruch 1, **dadurch gekennzeichnet, dass** die Dichte des Kompositpigments höher ist als die des organischen Pigments.

11. Verwendung gemäß Anspruch 1, **dadurch gekennzeichnet, dass** das organische Pigment ohne jedwede kovalente Bindungen an dem Mineralkern fixiert ist.

12. Verwendung gemäß einem der vorhergehenden Ansprüche, **dadurch gekennzeichnet, dass** die Zusammensetzung mindestens einen kosmetischen oder dermatologischen Wirkstoff umfasst.

13. Verwendung gemäß einem der vorhergehenden Ansprüche, **dadurch gekennzeichnet, dass** die Zusammensetzung mindestens eine Fettsubstanz, ein Wachs, einen Gummi oder ein filmbildendes Polymer umfasst.

14. Verwendung gemäß einem der vorhergehenden Ansprüche, **dadurch gekennzeichnet, dass** die Zusammensetzung in einer festen oder halbfesten Form vorliegt.

15. Verwendung gemäß einem der vorhergehenden Ansprüche, **dadurch gekennzeichnet, dass** die Zusammensetzung in flüssiger, pastenförmiger oder gellierter Form vorliegt.

16. Lippenstift, umfassend in einem physiologisch verträglichen Medium mindestens ein Kompositpigment, umfassend:
- einen Mineralkern, hergestellt aus Titanoxid,
- mindestens ein organisches Pigment, welches mindestens teilweise den Mineralkem bedeckt, und
- mindestens ein Bindemittel, welches aus der Gruppe ausgewählt ist, die besteht aus Silikonverbindungen, polymeren und oligomeren Verbindungen oder dergleichen, und insbesondere von Organosilanen, Fluoralkylorganosilanen und Polysiloxanen, und Kupplungsmitteln, basierend auf Silan, auf Titanaten, auf Aluminaten oder auf Zirkonaten, und Gemischen davon, zum Fixieren des organischen Pigments auf dem Mineralkem.

## Revendications

1. Utilisation d'une composition comprenant, dans un milieu physiologiquement acceptable, au moins un pigment composite comprenant :
- un noyau inorganique fait d'oxyde de titane,
- au moins un pigment organique recouvrant au moins partiellement le noyau inorganique, et
- au moins un liant choisi dans le groupe constitué par les composés siliconés, les composés polymériques, oligomériques ou similaires, et en particulier parmi les organosilanes, les organosilanes fluoroalkylés et les polysiloxanes, ainsi que divers agents couplants, tels que des agents couplants à base de silane, de titanates, d'aluminates, de zirconates et leurs mélanges, assurant la fixation du pigment organique sur le noyau inorganique.

2. Utilisation selon la revendication 1, **caractérisée en ce que** le pigment organique est choisi dans le groupe constitué par les pigments suivants (répertoriés selon leur Color Index) : pigment bleu : CI 42090, 69800, 69825, 73000, 74100, 74160 ; pigment jaune : CI 11680, 11710, 15985, 19140, 20040, 21100, 21108, 47000, 47005 ; pigment vert : CI 61565, 61570, 74260 ; pigment orange : CI 11725, 15510, 45370, 71105 ; pigment rouge : CI 12085, 12120, 12370, 12420, 12490, 14700, 15525, 15580, 15620, 15630, 15800, 15850, 15865, 15880, 17200, 26100, 45380, 45410, 58000, 73360, 73915, 75470 ; pigment noir : CI 77266.

3. Utilisation selon l'une quelconque des revendications précédentes, **caractérisée en ce que** le noyau inorganique est coloré.

4. Utilisation selon l'une quelconque des revendications précédentes, **caractérisée en ce que** la proportion massique du pigment composite dans la composition est comprise entre 0,1 et 20 %, notamment entre 0,1 et 15 % et mieux entre 0,5 et 10 % en poids par rapport au poids total de la composition.

5. Utilisation selon l'une quelconque des revendications précédentes, **caractérisée en ce qu'**elle est dépourvue de particules de TiO₂ non enrobées.

6. Utilisation selon l'une quelconque des revendications précédentes, **caractérisée en ce que** la taille des particules de pigment composite est comprise entre 0,001 et 150 µm, de préférence 0,01 et 50 µm, mieux entre 0,01 et 25 µm, mieux encore entre 0,05 et 10 µm.

7. Utilisation selon la revendication 6, **caractérisée en ce que** les particules de pigment composite ont une taille inférieure à 2 µm,

8. Utilisation selon la revendication 7, **caractérisée en ce que** les particules de pigment composite ont une taille inférieure ou égale à 1 µm.

9. Utilisation selon l'une quelconque des revendications précédentes, **caractérisée en ce que** la densité du noyau inorganique est supérieure à celle du pigment organique.

10. Utilisation selon la revendication 1, **caractérisée en ce que** la densité du pigment composite est supérieure à celle du pigment organique.

11. Utilisation selon la revendication 1, **caractérisée en ce que** le pigment organique est fixé au noyau inorganique sans aucune liaison covalente.

12. Utilisation selon l'une quelconque des revendications précédentes, **caractérisée en ce qu'**elle comprend au moins un actif cosmétique ou dermatologique.

13. Utilisation selon l'une quelconque des revendications précédentes, **caractérisée en ce qu'**elle comprend au moins un corps gras, une cire, une gomme ou un polymère filmogène.

14. Utilisation selon l'une quelconque des revendications précédentes, **caractérisée en ce qu'**elle se présente sous une forme solide ou semi-solide.

15. Utilisation selon l'une quelconque des revendications précédentes, **caractérisée en ce qu'**elle se présente sous forme liquide, pâteuse ou gélifiée.

16. Rouge à lèvres comprenant, dans un milieu physiologiquement acceptable, au moins un pigment composite comprenant :
- un noyau inorganique fait d'oxyde de titane,
- au moins un pigment organique recouvrant au moins partiellement le noyau inorganique, et
- au moins un liant choisi dans le groupe constitué par les composés siliconés, les composés polymériques, oligomériques ou similaires, et en particulier parmi les organosilanes, les organosilanes fluoroalkylés et les polysiloxanes, ainsi que divers agents couplants, tels que des agents couplants à base de silane, de titanates, d'aluminates, de zirconates et leurs mélanges, assurant la fixation du pigment organique sur le noyau inorganique.
